# FASCICULE DE BREVET EUROPEEN

(11) **EP 1 737 399 B1**
(45) Date de publication et mention de la délivrance du brevet: **03.12.2008**
(21) Numéro de dépôt: 05760051.2
(22) Date de dépôt: 21.04.2005
(51) Int. Cl.: A61F 5/00

(54) **Dispositif de cerclage gastrique motorisé**
Motorgesteuerte Magnetbandvorrichtung
Motor-operated gastric banding device

(30) Priorité: 21.04.2004 FR 0404223
(43) Date de publication de la demande: 03.01.2007
(73) Titulaire: Europlak, 83130 La Garde (FR)
(72) Inventeur: BUFFARD, Laurent, F-69005 Lyon (FR); VASSOR, Arnaud, F-74000 Annecy (FR)
(74) Mandataire: Bredema
(86) Numéro de dépôt international: PCT/FR2005/000995
(87) Numéro de publication internationale: WO 2005/105001

(56) Documents cités:
- EP-A- 0 876 808
- EP-A- 1 286 477
- FR-A- 2 756 485
- FR-A- 2 797 181
- US-A- 5 592 182
- US-A1- 2002 044 096
- US-B1- 6 475 136
- US-B1- 6 680 710

## Description

La présente invention concerne le secteur technique des AGM ou anneaux gastriques motorisés c'est-à-dire dont le diamètre de l'anneau peut être réglé par un micro-moteur embarqué.

On connaît des systèmes d'anneau gastrique du type comportant une chambre extérieure et un ballonnet intérieur disposé dans un liquide physiologique contenu dans l'espace entre la chambre et le ballonnet. Un plus ou moins grand gonflement du ballonnet augmente ou diminue le diamètre de l'anneau gastrique. L'adaptation du gonflement est effectuée à l'aide d'une seringue par laquelle le praticien envoie un liquide physiologique via une chambre de réception disposée sous une incision de la peau du patient, et un tube conduisant à l'anneau. On conçoit les inconvénients des telles solutions : douleurs au niveau de la chambre disposée à demeure, risques de fuites, abus de manipulations demandées à certaines occasions par les patients, etc..., risques d'infection.

Des appareils à anneau gastrique motorisé ont été développés, dont la commande est effectuée par télécommande sans avoir recours à des incisions, flux de liquide physiologique, chambre de réception, tubes etc...

On connaît notamment les brevets FR 2 816 828, EP 0015140, et FR 2 797 181 que l'on peut qualifier de brevet de base.

Ces systèmes comportent essentiellement un anneau non entièrement fermé, qui est destiné à être placé autour de l'oesophage, ledit anneau comportant une bande interne qui est fixée à une des extrémités de l'anneau et sort de l'anneau par son autre extrémité, ladite bande pouvant coulisser dans l'anneau sous l'effet d'une micro motorisation (système de traction) disposée dans un élément distinct comportant ladite motorisation, les moyens d'accrochage de ladite bande à ladite motorisation, et une électronique embarquée (électronique de récession de l'énergie, de traitement des signaux de commande, d'alimentation de la micro motorisation, d'envoi de signaux de commande à ladite motorisation, de gestion des mémoires et données mémoire, de la mise à jour de ces données, et autres fonctions de ce type).

Le document US 6 475 136 décrit un anneau gastrique motorisé, comportant un moteur agissant sur le diamètre de l'anneau, activé par induction vers une antenne réceptrice embarquée, à partir d'au moins une antenne émettrice extérieure. L'anneau comporte une antenne de réception et une électronique embarquée adaptée pour assurer une utilisation optimale de l'énergie reçue.

L'homme de métier aura compris que, en exerçant une traction sur la bande, on resserre l'anneau autour de l'oesophage, et inversement si on relâche la traction.

Ces divers appareils présentent un grand intérêt.

Cependant, un problème important se pose en ce sens que l'anneau gastrique peut se déplacer après sa pose, pour finalement reposer sur certains organes comme le foie ou l'estomac.

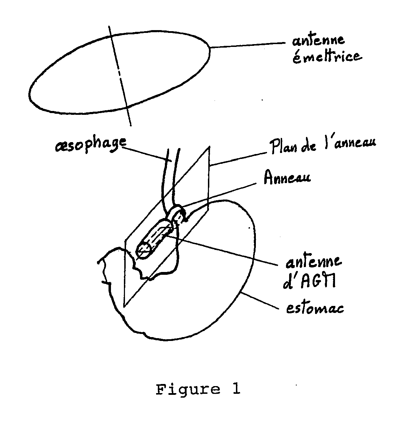

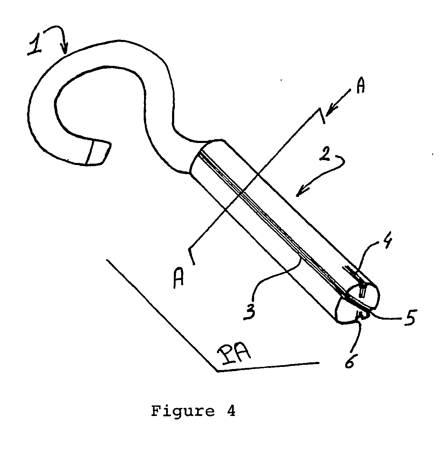

Pour préciser le mouvement subi par l'AGM, il est nécessaire de préciser les éléments suivants :
L'AGM est constitué (figure 4) d'un cylindre 2 contenant moteur, électronique et antenne réceptrice, et en avant de ce cylindre se trouve l'anneau 1 qui vient se placer autour de l'oesophage. Cet anneau dont le diamètre peut être augmenté ou diminué définit un plan dont l'orientation par rapport à l'antenne émettrice est capitale (figure 1).

En effet, cette orientation détermine la quantité d'énergie reçue par l'anneau. Une trop faible quantité d'énergie reçue se traduit d'abord par un arrêt de fonctionnement du moteur, puis par un arrêt de la communication avec l'AGM,

L'antenne des versions d'AGM de l'art antérieur est placée de manière standard à 90° du plan formé par l'anneau.

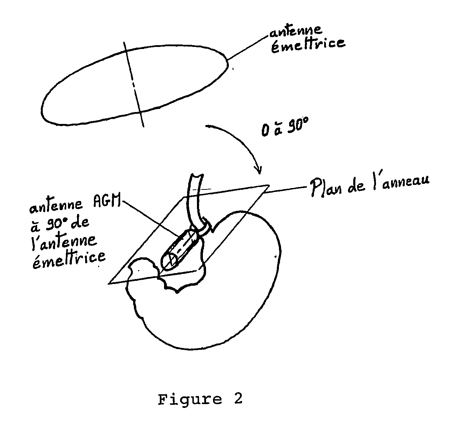

Le Demandeur a poursuivi ses études sur le sujet et a découvert que l'AGM se déplace dans le corps du patient après son implantation, ce qui n'était pas prévisible et est d'ailleurs aléatoire, ce qui confirme son imprévisibilité et le mérite du Demandeur, d'avoir orienté ses recherches dans ce sens. Le déplacement de l'AGM après implantation aboutit à une rotation qui, lorsqu'elle se produit, peut être très notable de l'anneau de l'AGM par rapport au plan de l'antenne extérieur. Cette rotation est en effet comprise entre 0° et 90° par rapport à l'orientation idéale de l'anneau (plan de l'anneau perpendiculaire au plan de l'antenne émettrice) (figure 2).

Compte tenu de ces rotations possibles l'antenne peut donc se trouver à un angle ne permettant pas de recevoir suffisamment d'énergie pour alimenter le moteur, et, dans certain cas, la communication n'est même plus possible.

Ceci peut éventuellement déboucher sur l'obligation de repositionner l'anneau, dans les cas extrêmes.

### Problème technique :

Le " problème technique " visé par ce brevet est de supprimer ce danger. Le problème technique « objectif » posé est donc d'imaginer un système qui assure, quel que soit le positionnement final de l'anneau gastrique motorisé (AGM), une réception qui permette le fonctionnement du moteur de l'AGM à une distance supérieure à 5 cm.

Naturellement, il est impossible d'augmenter suffisamment les dimensions de l'antenne extérieure émettrice, que l'on doit poser sur l'abdomen du patient, ni sa puissance ou sa durée d'émission (risques, entre autres, de brûlures par l'antenne au contact et risque d'échauffement interne de la zone corporelle où est implanté l'appareil) ; de même le choix de la fréquence d'émission n'est pas libre : la fréquence doit évidemment être très éloignée des fréquences des micro-ondes et doit au contraire correspondre à un rayonnement magnétique (sans effet notable sur le patient) et non électromagnétique.

Une solution est d'orienter l'antenne unique selon un angle " moyen " c'est-à-dire intermédiaire entre la position adoptée initialement (antenne à 90° de l'anneau) et une antenne dans le même plan que l'anneau. On place ainsi l'antenne selon un angle " moyen » de l'ordre de 45° par rapport à l'anneau.

Compte tenu des mouvements possibles de l'anneau motorisé après sa pose, on écarte ainsi le risque d'une réception nulle.

Selon un mode de réalisation particulier, l'invention consiste à adjoindre au moins une seconde antenne à la première, que celle-ci soit désorientée comme indiqué ci-dessus ou qu'elle soit orientée de manière standard comme dans l'art antérieur, ou toute solution intermédiaire. Cette seconde antenne (ou ces antennes supplémentaires) sera sensiblement à angle droit (ou à des angles régulièrement espacés en cas de plusieurs antennes) par rapport à la première antenne. Cette solution permet d'assurer que quel que soit l'angle de rotation de l'AGM une au moins des antennes permet une réception minimum d'énergie permettant d'alimenter le moteur de l'AGM.

### Résumé de l'invention :

L'invention telle que décrite dans la revendication 1 repose sur un anneau gastrique motorisé ou " AGM " du type comportant un moteur agissant sur le diamètre de l'anneau, activé par induction vers au moins une antenne réceptrice embarquée, à partir d'une antenne émettrice extérieure, caractérisé en ce que ledit anneau comporte au moins une antenne de réception, une des antennes au moins étant désorientée par rapport au plan dudit anneau gastrique, c'est-à-dire que l'antenne ne se trouve plus à angle droit du plan contenant l'anneau gastrique et une électronique embarquée adaptée pour assurer une utilisation optimale de l'énergie reçue.

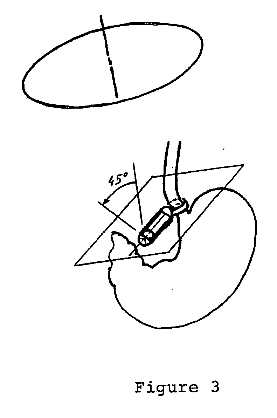

L'angle que l'antenne devra avoir par rapport au plan de l'anneau devra être d'au moins 20° et de préférence de l'ordre de 25 à 65° par rapport au plan de l'anneau. Cet angle sera trigonométriquement positif en regardant l'AGM par l'extrémité opposée à l'anneau (figure 3).

Toutefois, pour une désorientation de 45°, la puissance reçue par l'antenne réceptrice est de 70 % de la puissance reçue avec un angle de 0° entre antenne émettrice et antenne réceptrice.

Une telle perte de réception correspond à une diminution de la distance maximum de bon fonctionnement de l'AGM de 65 %.

Une amélioration avantageuse de la solution d'une antenne désorientée est de disposer de deux antennes et d'une électronique adaptée permettant d'utiliser au mieux l'énergie reçue par l'antenne la mieux orientée pour alimenter le moteur.

Dans cette configuration, la désorientation entre l'antenne émettrice et l'antenne réceptrice la mieux placée correspond à 22,5°.

L'invention concerne donc également un anneau gastrique motorisé caractérisé en ce que l'on dispose deux antennes décalées angulairement l'une par rapport à l'autre et dont au moins une est désorientée et une électronique adaptée permettant d'utiliser au mieux l'énergie reçue par l'antenne la mieux orientée pour alimenter le moteur.

Dans cette configuration, la désorientation entre l'antenne émettrice et l'antenne réceptrice la mieux placée correspond à 22,5°, si l'on suppose l'antenne émettrice placée sensiblement parallèlement à l'abdomen du porteur de l'AGM.

Pour cette différence d'orientation, l'énergie reçue par l'antenne la mieux orientée est de 92,4 %, ce qui correspond à une diminution de la distance maximum de bon fonctionnement de l'AGM de 21,1 %.

On notera que ce genre de solution se heurtait à deux obstacles importants : d'une part, plus d'une antenne signifie un encombrement plus important et une complexité plus grande, alors que le volume disponible et le poids autorisé ne sont pas extensibles à volonté ; d'autre part, deux antennes, ou plus de deux antennes, risquent de voir leurs signaux interférer, se combattre jusqu'à éventuellement s'annihiler totalement.

L'invention a résolu ces deux problèmes.

### Description détaillée de l'invention :

L'invention concerne donc un anneau gastrique motorisé caractérisé en ce qu'il comporte au moins une antenne à orientation angulaire décalée d'au moins 20° par rapport au plan de l'anneau, de préférence au moins deux antennes à décalage angulaire de réception de l'énergie destinée à la motorisation et à l'électronique embarquée correspondante, énergie fournie par induction par au moins une antenne extérieure, lesdites au moins deux antennes de réception étant décalées angulairement l'une par rapport à l'autre, et en ce qu'il comporte une électronique embarquée complémentaire apte à utiliser les énergies reçues individuellement par chaque antenne.

Selon un mode de réalisation particulier, l'invention concerne un anneau gastrique motorisé caractérisé en ce qu'il comporte au moins deux antennes à décalage angulaire de réception de l'énergie destinée à la motorisation et à l'électronique embarquée correspondante, énergie fournie par induction par au moins une antenne extérieure, lesdites au moins deux antennes de réception étant décalées angulairement l'une par rapport à l'autre, et en ce qu'il comporte une électronique embarquée complémentaire apte à utiliser les énergies reçues individuellement par chaque antenne.

Selon encore un mode de réalisation particulier, ledit anneau comportant au moins deux antennes, et notamment deux antennes, est caractérisé en ce qu'il comporte au moins une antenne à orientation angulaire décalée d'au moins 20° par rapport au plan de l'anneau.

Selon un mode de réalisation particulier, l'AGM comporte deux antennes.

Selon encore un mode de réalisation particulier, lesdites deux antennes sont décalées angulairement de 90° l'une par rapport à l'autre.

Selon encore un mode de réalisation particulier, lesdites deux antennes sont dans des plans dont l'intersection forme l'axe longitudinal de la partie motorisation et électronique de l'AGM.

Avantageusement, ledit anneau gastrique comporte une pluralité d'antennes décalées angulairement l'une par rapport à l'autre de 45° à 135°.

Selon une option, ledit AGM comporte trois ou quatre antennes décalées angulairement de manière régulière.

Selon un mode de réalisation particulier, les antennes sont constituées par des bobinages entourant au moins une partie du corps de " motorisation-électronique embarquée ", de préférence dans des canaux ou encoches périphériques ménagés à la surface dudit corps.

D'autres caractéristiques et avantages de l'invention seront mieux compris à la lecture de la description qui va suivre, et en se référant au dessin annexé sur lequel :
- la figure 1 représente un anneau gastrique motorisé mis en place, autour de l'oesophage avec une antenne à 90° du plan de l'anneau, et une antenne émettrice ;
- la figure 2 représente un AGM ayant subi une modification de position dans l'abdomen, en général comprise entre 0 et 90°, ce qui fait pivoter d'autant l'antenne par rapport à l'antenne émettrice
- la figure 3 représente une solution non limitative selon l'invention comportant une antenne réceptrice « désorientée » de 45° par rapport au plan de l'anneau, dans la configuration de la figure 2
- la figure 4 représente un AGM du type considéré, en perspective schématique, avec sa partie " anneau " proprement dite 1 et sa partie ou corps "motorisation - électronique embarquée " 2 (dont les antennes 3 et 4 décalées angulairement l'une par rapport à l'autre correspondant à une version de l'invention, non limitative)

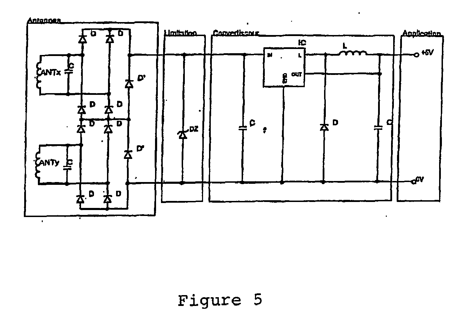

- la figure 5 représente un schéma électrique de couplage de deux antennes, extrapolable à plus de deux antennes :
   - ANT: x ou respectivement y : antenne selon l'axe x ou respectivement y ;
   - C: condensateur
   - D: diode
   - DZ: diode Zener
   - L: Inductance
   - IC: circuit intégré (permet de convertir la tension à un voltage approprié, notamment 5V pour l'application AGM, pour alimenter le moteur et son circuit de commande)

L'invention concerne encore les AGM du type décrit ci-dessus comportant un schéma électrique de couplage d'antenne tel que décrit dans la section "procécé " ci-dessus.

L'invention concerne encore toutes les applications du procédé de couplage d'antennes tel que décrit ci-dessus à des AGM de tous types, notamment dont le système de motorisation serait différent (pincement / rotation de deux branches de l'anneau au lieu de traction sur une bande, etc....)

### EXEMPLES

On a construit un prototype selon l'invention dont les caractéristiques sont les suivantes :
Antenne 3 désorientée de 45° par rapport au plan de l'anneau PA, constituée de 100 spires bobinées sur un cadre de longueur par largeur comprenant une gorge en U de section 1,7 x 2 mm.
Antenne 4 à 90° de l'antenne 3, constituée de 120 spires bobinées sur un cadre de longueur 75mm par largeur 11 mm comprenant une gorge en U de section 1,7 x 2 mm

En utilisant une antenne émettrice de diamètre 300mm, alimentée sous 1000V et 0,3A, ce type de dispositif permet d'obtenir le fonctionnement d'un moteur sous 40 mA et 5V à partir d'une tension de 12V en sortie de limiteur de tension, ceci à une distance de 15cm.

## Revendications

1. Anneau gastrique motorisé (1) ou "AGM " du type comportant :
- au moins une antenne réceptrice embarquée ;
- un moteur agissant sur le diamètre de l'anneau, activé par induction, à partir au moins d'une antenne émettrice extérieure vers l'antenne réceptrice embarquée, ; et
- une électronique embarquée (2) adaptée pour assurer une utilisation optimale de l'énergie reçue ;
ledit anneau étant **caractérisé en ce que** au moins une antenne de réception (3, 4) est fixée audit anneau gastrique et désorientée par rapport au plan dudit anneau gastrique, selon un angle supérieur ou égal à 20°, cet angle étant trigonométriquement positif en regardant l'AGM par l'extrémité opposée à l'anneau.

2. Anneau gastrique motorisé (1) selon la revendication 1, **caractérisé en ce que** ladite antenne désorientée (3) est écartée par rapport au plan de l'anneau gastrique selon un angle compris entre 25 et 65°, et de préférence selon un angle de 45°.

3. Anneau gastrique motorisé (1) selon la revendication 1 ou la revendication 2, **caractérisé en ce qu'**il comprend deux antennes décalées angulairement (3, 4) l'une par rapport à l'autre et dont au moins une est désorientée et une électronique adaptée permettant d'utiliser au mieux l'énergie reçue par l'antenne la mieux orientée pour alimenter le moteur.

4. Anneau gastrique motorisé (1) selon la revendication 3, **caractérisé en ce que** lesdites deux antennes réceptrices sont décalées angulairement de 90° l'une par rapport à l'autre.

5. Anneau gastrique motorisé (1) selon la revendication 3 ou 4 **caractérisé en ce que** lesdites antennes réceptrices sont dans des plans dont l'intersection forme l'axe longitudinal de la partie motorisation et électronique dudit anneau gastrique.

6. Anneau gastrique motorisé (1) selon l'une des revendications 1 à 2, **caractérisé en ce qu'**il comporte une pluralité d'antennes décalées angulairement l'une par rapport à l'autre de 45° à 135°.

7. Anneau gastrique motorisé (1) selon la revendication 76, **caractérisé en ce qu'**il comporte trois ou quatre antennes décalées angulairement de manière régulière.

8. Anneau gastrique motorisé (1) selon l'une quelconque des revendications 3 à 7, **caractérisé en ce que** les antennes sont constituées par des bobinages entourant au moins une partie du corps de " motorisation-électronique embarquée " (2), de préférence dans des canaux ou encoches périphériques ménagés à la surface dudit corps.

9. Anneau gastrique motorisé (1) selon l'une quelconque des revendications 3 à 8, **caractérisé en ce qu'**il comporte un circuit électrique de couplage de deux antennes réceptrices, extrapolable à plus de deux antennes, comportant au moins les éléments suivants :
- une antenne ANT x selon l'axe x et une antenne ANT y selon l'axe Y ;
- un condensateur C ;
- une diode D ;
- unde diode Zener Dz ;
- une inductance L ; et
- un circuit intégré permettant de convertir la tension à un voltage approprié pour alimenter le moteur et son circuit de commande.

10. Anneau gastrique motorisé (1) selon l'une quelconque des revendications 3 à 9, **caractérisé en ce qu'**il comprend :
- une antenne (3) désorientée de 45° par rapport au plan de l'anneau PA, constituée de 100 spires bobinées sur un cadre de longueur par largeur comprenant une gorge en U de section 1,7 x 2 mm, et
- antenne (4) à 90° de l'antenne 3, constituée de 120 spires bobinées sur un cadre de longueur 75mm par largeur 11 mm comprenant une gorge en U de section 1,7 x 2 mm,
ces antennes étant aptes à coopérer avec une antenne émettrice de diamètre 300mm, alimentée sous 1000V et 0,3A, ce type de dispositif permettant d'obtenir le fonctionnement d'un moteur sous 40 mA et 5V à partir d'une tension de 12V en sortie de limiteur de tension, ceci à une distance de 15cm.

## Claims

1. A motorized gastric band (1) or "MGB" of the type including:
- at least one on-board receiving antenna;
- one motor acting on the diameter of the band, activated by induction from at least one outer emitting antenna to the on-board receiving antenna; and
- an on-board electronic (2) adapted for providing the optimum utilization of the received energy;
said band being **characterized in that** the at least one receiving antenna (3, 4) is attached to said gastric band and de-oriented with respect to the plane of said gastric band, according to an angle equal to or greater than 20°, such angle being positive from a trigonometrical point of view, when viewing the MGB from the end opposite the band.

2. A motorized gastric band (1) according to claim 1, **characterized in that** said de-oriented antenna (3) is positioned away from the gastric band plane according to an angle between 25 and 65° and preferably according to an angle of 45°.

3. A motorized gastric band (1) according to claim 1 or claim 2, **characterized in that** it includes two antennas (3, 4) angularly shifted with respect to each other and at least one of which is de-oriented, and an adapted electronic making it possible to use at best the energy received by the best oriented antenna, in order to power the motor.

4. A motorized gastric band (1) according to claim 3, **characterized in that** said two receiving antennas are angularly shifted by 90° with respect to each other.

5. A motorized gastric band (1) according to claim 3 or 4, **characterized in that** said receiving antennas are positioned in planes the intersection of which forms the longitudinal axis of the motorization and electronic part of said gastric band.

6. A motorized gastric band (1) according to any one of claims 1 to 2, **characterized in that** it includes a plurality of antennas angularly shifted by 45° to 135° with respect to each other.

7. A motorized gastric band (1) according to claim 6, **characterized in that** it includes three or four antennas angularly shifted in a regular way.

8. A motorized gastric band (1) according to any one of claims 3 to 7, **characterized in that** the antennas are composed of windings surrounding at least a part of the "on-board motorization-electronic" body (2), preferably in peripheral notches or channels arranged at the surface of said body.

9. A motorized gastric band (1) according to any one of claims 3 to 8, **characterized in that** it includes an electric circuit for the coupling of two receiving antennas, which can be scaled to more than two antennas, including at least the following elements:
- one antenna ANT x along axis x and one antenna ANT y along axis y;
- one condenser C;
- one diode D;
- one Zener diode Dz;
- one inductor L; and
- one integrated circuit making it possible to convert the voltage to a voltage adapted to the powering of the motor and the control circuit thereof.

10. A motorized gastric band (1) according to any one of claims 3 to 9, **characterized in that** it includes:
- an antenna de-oriented by 45° with respect to the band PA plane, composed of 100 turns wound on a frame in length in width, comprising a U-shaped groove 1.7 x 2mm in section, and
- an antenna (4) at 90° with the antenna (3), composed of 120 turns wound on a frame 75mm in length x 11mm in width, comprising a U-shaped groove 1.7 x 2mm in section,
such antennas being able to cooperate with an emitting antenna 300mm in diameter, powered with 1000V and 0.3A, such type of device making it possible to obtain the operation of a motor with 40mA and 5V from a 12V voltage at the output of the voltage limiter, from a distance of 15cm.

## Patentansprüche

1. Motorisierter Magenring (1) oder "AGM" bestehend aus:
- mindestens einer eingebauten Empfangsantenne;
- einem auf den Durchmesser des Rings wirkenden Motor, der per Induktion aktiviert wird, ab mindestens einer externen Sendeantenne zu einer eingebauten Empfangsantenne; und
- einer eingebauten Elektronik (2), die eine optimale Nutzung der aufgenommenen Energie gewährleisten kann;
wobei der genannte Ring **dadurch gekennzeichnet ist, dass** die mindestens eine Empfangsantenne (3, 4) am genannten Magenring befestigt und in Bezug auf die Ebene des genannten Magenrings gemäß einem Winkel von größer oder gleich 20° weggerichtet ist, wobei dieser Winkel trigonometrisch positiv ist, wenn der AGM vom dem Ring gegenüber gerichteten Ende aus betrachtet wird.

2. Motorisierter Magenring (1) gemäß Anspruch 1, **dadurch gekennzeichnet, dass** die genannte weggerichtete Antenne (3) in Bezug auf die Ebene des Magenrings gemäß einem Winkel zwischen 25 und 65° und vorzugsweise einem Winkel von 45° abgespreizt ist.

3. Motorisierter Magenring (1) gemäß Anspruch 1 oder Anspruch 2, **dadurch gekennzeichnet, dass** er zwei winkelförmig zueinander versetzte Antennen (3, 4) umfasst, von denen mindestens eine weggerichtet ist, und eine entsprechende Elektronik, die es ermöglicht, die von der am Besten ausgerichteten Antenne aufgenommene Energie optimal zu nutzen, um den Motor zu versorgen.

4. Motorisierter Magenring (1) gemäß Anspruch 3, **dadurch gekennzeichnet, dass** die beiden genannten Empfangsantennen (3, 4) in einem Winkel von 90° zueinander versetzt sind.

5. Motorisierter Magenring (1) gemäß Anspruch 3 oder 4, **dadurch gekennzeichnet, dass** sich die genannten Empfangsantennen in Ebenen befinden, deren Überschneidung die Längsachse des Teils Motorisierung und Elektronik des genannten Magenrings bildet.

6. Motorisierter Magenring (1) gemäß einem der Ansprüche 1 bis 2, **dadurch gekennzeichnet, dass** er eine Mehrzahl von Antennen umfasst, die in einem Winkel von 45° bis 135° zueinander versetzt sind.

7. Motorisierter Magenring (1) gemäß Anspruch 6, **dadurch gekennzeichnet, dass** er drei oder vier Antennen umfasst, die in einem regelmäßigen Winkel versetzt sind.

8. Motorisierter Magenring (1) gemäß einem der Ansprüche 3 bis 7, **dadurch gekennzeichnet, dass** die Antennen aus Wicklungen bestehen, die mindestens einen Teil des Körpers "Motorisierung - eingebaute Elektronik" (2) umgeben, vorzugsweise in umlaufenden Kanälen oder Kerben, die an der Oberfläche des genannten Körpers vorgesehen sind.

9. Motorisierter Magenring (1) gemäß einem der Ansprüche 3 bis 8, **dadurch gekennzeichnet, dass** er eine elektronische Kopplungsschaltung von zwei Empfangsantennen umfasst, die auf mehr als zwei Antennen erweitert werden kann, mit mindestens einem der folgenden Elemente:
- eine Antenne ANT x gemäß der x-Achse und eine Antenne y gemäß der y-Achse;
- einen Kondensator C;
- eine Diode D;
- eine Zener-Diode Dz;
- eine Induktanz L; und
- einen integrierten Schaltkreis für die Umwandlung der Spannung in eine für die Versorgung des Motors und seiner Steuerschaltung geeignete Spannung.

10. Motorisierter Magenring (1) gemäß einem der Ansprüche 3 bis 9, **dadurch gekennzeichnet, dass** er umfasst:
- eine Antenne (3), die um 45° in Bezug auf die Ebene des Rings PA weggerichtet ist und aus 100 Windungen besteht, die um einen Rahmen von Länge mal Breite mit einem U-förmigen Hals mit einem Querschnitt von 1,7 x 2 mm gewickelt sind, und
- eine Antenne (4) um 90° von Antenne 3, bestehend aus 120 Windungen, die um einen Rahmen von 75 mm Länge mal 11 mm Breite mit einem U-förmigen Hals mit einem Querschnitt von 1,7 x 2 mm gewickelt sind,
wobei diese Antennen mit einer Sendeantenne mit einem Durchmesser von 300 mm, versorgt mit 1000 V und 0,3 A, zusammenwirken können, wobei dieser Vorrichtungstyp ermöglicht, einen Motor mit 40 mA und 5 V ab einer 12V-Spannung am Ausgang eines Strombegrenzers zu betreiben, und zwar aus einer Entfernung von 15 cm.
